(19) Europäisches Patentamt

European Patent Office

Office européen des brevets

(11) **EP 1 029 926 A1**

(12) **EUROPEAN PATENT APPLICATION**

(43) Date of publication:
23.08.2000 Bulletin 2000/34

(21) Application number: 00102645.9

(22) Date of filing: 08.02.2000

(51) Int. Cl.[7]: **C12P 19/32**, C12N 15/63,
C12N 15/52, C12N 15/54,
C12N 15/55, C12N 9/00,
C12N 9/12, C12N 9/16,
C12N 1/21
// (C12N1/21, C12R1:15)

(84) Designated Contracting States:
**AT BE CH CY DE DK ES FI FR GB GR IE IT LI LU MC NL PT SE**
Designated Extension States:
**AL LT LV MK RO SI**

(30) Priority: 08.02.1999 JP 2973899

(71) Applicant:
**KYOWA HAKKO KOGYO CO., LTD.**
**Chiyoda-ku, Tokyo 100-8185 (JP)**

(72) Inventors:
• **Takano, Yukata,**
c/o Kyowa Hakko Kogyo Co.,Ltd.
**Hofu-shi, Yamaguchi (JP)**
• **Ikeda, Masato,**
c/o Kyowa Hakko Kogyo Co.,Ltd.
**Hofu-shi, Yamaguchi (JP)**
• **Fujio, Tatsuro,**
c/o Kyowa Hakko Kogyo Co.,Ltd.
**Tokyo (JP)**

(74) Representative:
**VOSSIUS & PARTNER**
**Siebertstrasse 4**
**81675 München (DE)**

(54) **Process for producing purine nucleotides**

(57)    The present invention provides a process for producing a purine nucleotide which comprises: culturing in a medium a microorganism having the ability to produce a precursor of the purine nucleotide and carrying an introduced DNA which can induce and express an enzyme capable of synthesizing the purine nucleotide from said precursor; allowing said precursor of the purine nucleotide to accumulate in the culture; inducing and expressing the enzyme capable of synthesizing the purine nucleotide from said precursor; allowing the purine nucleotide formed from said precursor to accumulate in said culture; and then recovering said purine nucleotide therefrom. Microorganisms useful in said process are also provided.

Fig. 1

EP 1 029 926 A1

SEQUENCE LISTING

<110> KYOWA HAKKO KOGYO CO., LTD.

<120> Process for producing purine nucleotides

<130> E 1166 EP

<140>
<141>

<150> JP 29738/99
<151> 1999-02-08

<160> 4

<170> PatentIn Ver. 2.1

<210> 1
<211> 47
<212> DNA
<213> Artificial Sequence

<220>
<223> Description of Artificial Sequence: synthetic, no
      natural origin

<400> 1
ccgcggctta aggaagttac ctgtgtgact caacctgcaa caactcc          47

<210> 2
<211> 40
<212> DNA
<213> Artificial Sequence

<220>
<223> Description of Artificial Sequence: synthetic, no
      natural origin

<400> 2
aaaggatcct agaagtttta ctcccactcg atggttcccg               40

<210> 3
<211> 47
<212> DNA
<213> Artificial Sequence

<220>
<223> Description of Artificial Sequence: synthetic, no
      natural origin

<400> 3
ccgcggctta aggaagtgac tttgatgaaa tttcccggta aacgtaa          47

<210> 4
<211> 40
<212> DNA
<213> Artificial Sequence

```
<220>
<223> Description of Artificial Sequence: synthetic, no
      natural origin

<400> 4
aaaggatcca cgataactta acgatcccag taagactctt                    40
```

**Description**

Background of the Invention

The present invention relates to a process for producing purine nucleotides which are in great demand as seasoning agents, and to microorganisms useful in said process.

5'-Guanylic acid (hereinafter abbreviated as GMP) and 5'-inosinic acid (hereinafter abbreviated as IMP) are purine nucleotides which exhibit a strong flavor-enhancing activity and are widely used as chemical seasoning components.

Some processes are known for producing these flavor-enhancing nucleotides: for example, a process which comprises hydrolyzing RNA extracted from yeast cells with ribonuclease P1 and then isolating and purifying the desired 5'-nucleotide [Food Technol., 18, 287 (1964)]; a process of directly producing IMP by fermentation using a microorganism having the ability to produce IMP [Agricultural and Biological Chemistry, 46, 2257 (1982)]; a process which comprises converting nucleosides such as inosine and guanosine into nucleotides by chemical phosphorylation [Bulletin of the Chemical Society of Japan, 42, 3505 (1969) ]; a process which comprises producing 5-amino-4-imidazolecarboxamide riboside (hereinafter abbreviated as AICAR) by fermentation using a mutant of Bacillus megaterium, and then producing a nucleotide from AICAR by chemical synthesis [Biotechnol. Bioeng., 9, 329 (1967); J. Org. Chem., 32, 1825 (1967)]; and a process which comprises culturing a mutant of Corynebacterium ammoniagenes which produces 5'-xanthylic acid (hereinafter abbreviated as XMP) in a medium to form and accumulate XMP, culturing Escherichia coli increased in the activity of XMP aminase (also known as GMP synthetase) obtained by self-cloning, and then producing GMP from XMP by enzyme reaction using the cultured E. coli cells as an enzyme source [Biosci. Biotech. Biochem., 61, 840 (1997); Japanese Published Unexamined Patent Application No. 233798/88].

To sum up, processes for producing flavor-enhancing nucleotides so far developed can be classified into the following three groups in principle [Shoichi Takao, et al. (ed.), Oyo Biseibutsugaku (Applied Microbiology), Buneido Shuppan (1996)]:

(1) a method in which yeast RNA is degraded with a microorganism-derived enzyme or chemically (the RNA degradation method),
(2) a method of directly producing a nucleotide by culturing a mutant of a microorganism in a medium containing sugars, a nitrogen source and a phosphoric acid source (the direct fermentation method), and
(3) a method which comprises producing an intermediate for the nucleotide synthesis by fermentation, and converting said intermediate into a nucleotide chemically or enzymatically (the method which is a combination of fermentation and chemical synthesis or enzymatic conversion).

In the RNA degradation method (method (1) above), pyrimidine nucleotides are also produced in almost the same amounts as flavor-enhancing purine nucleotides, and so the purification step necessarily becomes complicated. In the direct fermentation method (method (2) above), it is not easy to breed a microorganism which can extracellularly form and accumulate a considerable amount of nucleotides because of low membrane permeability of nucleotides, and thus it is difficult to obtain an economically satisfactory productivity of nucleotides. Specifically, there has not been known a process for direct production of GMP so far. However, it is known that XMP can be exceptionally formed and accumulated in considerable amounts [Shoichi Takao, et al. (ed.), Oyo Biseibutsugaku (Applied Microbiology), Buneido Shuppan (1996)]. At present, as an economically advantageous method for producing flavor-enhancing nucleotides, the combination method of fermentation and chemical synthesis or enzymatic conversion (method (3)) is widely employed for industrial production of the nucleotides.

In this combination method, one of the points for the improvement of the entire productivity is to enhance the yield of an intermediate for the nucleotide synthesis in the fermentation step (the first stage of the process). To this end, a large number of microorganisms having high productivity of XMP, guanosine or inosine have been bred [Agricultural and Biological Chemistry, 42, 399 (1978); Agricultural and Biological Chemistry, 43, 1739 (1979); Agricultural and Biological Chemistry, 46, 2347 (1982)].

There have also been developed various methods of chemical or enzymatic conversion of an intermediate into a nucleotide (the second stage of the process). As a chemical method, site-specific phosphorylation reaction

(nucleoside →5'-nucleotide)

[Bulletin of the Chemical Society of Japan, 42, 3505 (1969)] is industrially used. However, the chemical phosphorylation reaction requires the intermediate step to purify a nucleoside as a phosphate group acceptor to the necessary extent and also requires the use of a chemical reaction vessel in addition to a fermentor used for the fermentation step. Therefore, the enzymatic methods using aminase or phosphorylase attract attention and have been studied. As for the amination, a method using XMP aminase is known [Shoichi Takao, et al. (ed.), Oyo Biseibutsugaku (Applied Microbiology), Buneido Shuppan (1996)].

As for the phosphorylation, methods using phosphotransferase, kinase and phosphatase are known. In particular, the reaction utilizing kinase or phosphatase has been studied as an efficient method. For example, there have been developed a process for producing a 5'-nucleotide by the use of an Escherichia coli strain carrying a gene encoding inosine-guanosine kinase of Escherichia coli (WO91/08286), a process for producing a 5'-nucleotide by the use of a Corynebacterium ammoniagenes strain carrying a gene encoding inosine-guanosine kinase of Exiguobacterium acetylicum (WO96/30501), and a process for producing a 5'-nucleotide by the use of an Escherichia coli strain carrying a gene prepared by imparting a random mutation to the phosphatase gene of Morganella morganii (Japanese Published Unexamined Patent Application No. 37785/97, Japanese Published Unexamined Patent Application No. 201481/98).

As the phosphate group donor, various compounds have been studied. For example, methods using the following substances are known: P-nitrophenyl phosphate (Japanese Published Examined Patent Application No. 2985/64), inorganic phosphoric acid (Japanese Published Examined Patent Application No. 1186/67, Japanese Published Examined Patent Application No. 44350/74), polyphosphoric acid (Japanese Published Unexamined Patent Application No. 56390/78), acetyl phosphate (Japanese Published Unexamined Patent Application No. 82098/81), ATP (Japanese Published Unexamined Patent Application No. 230094/88), polyphosphoric acid, phenyl phosphate and carbamyl phosphate (Japanese Published Unexamined Patent Application No. 37785/97), and pyrophosphoric acid (Japanese Published Unexamined Patent Application No. 37785/97, Japanese Published Unexamined Patent Application No. 201481/98).

However, these methods are not industrially advantageous for the reasons that the substrates to be used are expensive or unstable, the purification steps become complicated because of formation of reaction by-products, etc. As a practical method to meet the economical need, the nucleotide production system by the use of the ATP-regenerating system has been developed. In this system, a microorganism regenerates ATP from AMP or ADP using inorganic phosphoric acid in the course of carbohydrate metabolism. For example, a method has been proposed in which the activity to biosynthesize ATP by metabolizing glucose possessed by an XMP-producing microorganism is used as the ATP-regenerating system. There are publications teaching a method for producing GMP using inexpensive glucose and inorganic phosphoric acid as ATP-regenerating substrates instead of ATP by coupling the above ATP-regenerating system with the XMP aminase activity of a microorganism having the ability to form GMP from XMP and ATP [Biosci. Biotech. Biochem., 61, 840 (1997)], and a similar method using inosine kinase as an enzyme to produce IMP (Japanese Published Unexamined Patent Application No. 230094/88).

Two kinds of microorganisms are used in a process for producing GMP or IMP which comprises the fermentation step to produce XMP, guanosine or inosine by direct fermentation and the reaction step to enzymatically convert the fermentation product into GMP or IMP.

That is, in the fermentation step which is the first stage of the process, a mutant bred as an XMP-, guanosine- or inosine-producing microorganism (a producing microorganism) is used. In the reaction step of amination or phosphorylation which is the second stage of the process, a microorganism carrying a highly expressed gene encoding a protein having XMP aminase activity or inosine-guanosine kinase activity (a converting microorganism) is used.

ATP which is necessary in the reaction of the second stage is regenerated by the ATP-regenerating activity of not only the converting microorganism used in the second stage, but also the producing microorganism used in the first stage.

Thus, the reaction step of the second stage is a system coupling the XMP aminase activity or the inosine-guanosine kinase activity and the ATP-regenerating activity of two microorganisms.

The outline of the whole process is described below.

First, a producing microorganism is cultured in a medium mainly comprising sugars and nitrogen sources in a large fermentor to form and accumulate XMP, guanosine or inosine. Separately, a converting microorganism is cultured in a small fermentor. When the fermentation of the first stage is completed, the separately cultured converting microorganism is added to the large fermentor to carry out phosphorylation reaction or amination reaction in the presence of an inexpensive energy donor and a phosphate group donor.

The above process utilizing the enzyme reaction is advantageous compared with the chemical phosphorylation method as described above. However, it is still not entirely satisfactory in respect of efficiency because a small fermentor is necessary for culturing a converting microorganism, and the amount of the medium must be reduced at the fermentation step of the first stage in view of the addition of a converting microorganism, which lowers the amount of the desired nucleotide obtained per batch.

An object of the present invention is to develop a process for producing a purine nucleotide efficiently in one fermentor.

Summary of the Invention

The present inventors pursued the possibility of developing a more excellent process for producing a purine nucleotide in which fermentation to produce a precursor of the nucleotide and reaction to convert said precursor into the nucleotide can be carried out successively using only one microorganism in one fermentor, by introducing a gene of an enzyme capable of synthesizing the purine nucleotide from its precursor into a fermentation microorganism having the ability to produce the precursor of the purine nucleotide and by regulating the expression of the gene in both of the fermentation step and the reaction step. As a result of intensive studies in the pursuit of this possibility, the present invention has been completed.

The present invention relates to the following (1)-(20) .

(1) A process for producing a purine nucleotide which comprises: culturing in a medium a microorganism having the ability to produce a precursor of the purine nucleotide and carrying an introduced DNA which can express an enzyme capable of synthesizing the purine nucleotide from said precursor upon induction; allowing said precursor of the purine nucleotide to accumulate in the culture; inducing the expression of the enzyme capable of synthesizing the purine nucleotide from said precursor and expressing said enzyme; allowing the purine nucleotide formed from said precursor to accumulate in said culture; and then recovering said purine nucleotide therefrom.

(2) The process according to the above (1), wherein the precursor of the purine nucleotide is 5'-xanthylic acid, the enzyme capable of synthesizing the purine nucleotide from said precursor is 5'-xanthylic acid aminase, and the purine nucleotide is 5'-guanylic acid.

(3) The process according to the above (1), wherein the precursor of the purine nucleotide is guanosine, the enzyme capable of synthesizing the purine nucleotide from said precursor is inosine-guanosine kinase or phosphatase, and the purine nucleotide is 5'-guanylic acid.

(4) The process according to the above (1), wherein the precursor of the purine nucleotide is inosine, the enzyme capable of synthesizing the purine nucleotide from said precursor is inosine-guanosine kinase or phosphatase, and the purine nucleotide is 5'-inosinic acid.

(5) The process according to (1) to (4) above, wherein the microorganism belongs to the genus selected from the group consisting of Corynebacterium, Escherichia and Bacillus.

(6) The process according to the above (5), wherein the microorganism is Corynebacterium ammoniagenes.

(7) The process according to the above (6), wherein the microorganism is Corynebacterium ammoniagenes ATCC 6872/pLAC857 (FERM BP-6639) or Corynebacterium ammoniagenes ATCC 6872/pIGK2 (FERM BP-6638).

(8) The process according to (1) to (7), above which is characterized in that the expression of the enzyme capable of synthesizing the purine nucleotide is induced by the change of condition selected from the group consisting of rise in temperature, rise in pH and rise in osmotic pressure, or by the change of the carbon source from sugars to non-sugars.

(9) The process according to the above (8), wherein the non-sugar carbon source is acetic acid or acetate.

(10) A DNA which can express an enzyme capable of synthesizing a purine nucleotide from its Precursor upon induction.

(11) The DNA according to the above (10), which can express the enzyme capable of synthesizing the purine nucleotide upon induction by the change of condition selected from the group consisting of rise in temperature, rise in pH and rise in osmotic pressure, or by the change of the carbon source from sugars to non-sugars.

(12) The DNA according to the above (11), wherein the non-sugar carbon source is acetic acid or acetate.

(13) The DNA according to (10) to (12) above, which is the plasmid DNA contained in the microorganism deposited under the deposit number FERM BP-6639 or FERM BP-6638.

(14) A microorganism having the ability to produce a precursor of a purine nucleotide and carrying an introduced DNA which can express an enzyme capable of synthesizing the purine nucleotide from said precursor upon induction.

(15) The microorganism according to the above (14), wherein the precursor of the purine nucleotide is 5'-xanthylic acid, the enzyme capable of synthesizing the purine nucleotide from said precursor is 5'-xanthylic acid aminase, and the purine nucleotide is 5'-guanylic acid.

(16) The microorganism according to the above (14), wherein the precursor of the purine nucleotide is guanosine, the enzyme capable of synthesizing the purine nucleotide from said precursor is inosine-guanosine kinase orphosphatase, and the purine nucleotide is 5'-guanylic acid.

(17) The microorganism according to the above (14), wherein the precursor of the purine nucleotide is inosine, the enzyme capable of synthesizing the purine nucleotide from said precursor is inosine-guanosine kinase o_phosphatase, and the purine nucleotide is 5'-inosinic acid.

(18) The microorganism according to the above (14), which belongs to the genus selected from the group consisting of Corynebacterium, Escherichia and Bacillus.

(19) The microorganism according to the above (14), which is <u>Corynebacterium</u> <u>ammoniagenes</u>.

(20) The microorganism according to the above (19), which is <u>Corynebacterium</u> <u>ammoniagenes</u> ATCC 6872/pLAC857 (FERM BP-6639) or <u>Corynebacterium</u> <u>ammoniagenes</u> ATCC 6872/pIGK2 (FERM BP-6638).

<u>Brief Description of the Drawings</u>

Fig. 1 is a drawing showing the structure of plasmid pLAC857.
Fig. 2 is a drawing showing the structure of plasmid pGUA2.
Fig. 3 is a drawing showing the structure of plasmid pIGK2.

The symbols used in the drawings represent the following.

| | |
|---|---|
| $P_L$: | $P_L$ promoter |
| guaA: | XMP aminase gene derived from <u>Escherichia</u> <u>coli</u> |
| C.glt ORI: | Replication origin of <u>Corynebacterium</u> <u>glutamicum</u> |
| Spc$^r$: | Spectinomycin resistance gene |
| cI857: | Temperature sensitive repressor gene |
| $P_{ICL}$: | Expression-regulating region of the isocitrate lyase gene derived from <u>Corynebacterium</u> <u>glutamicum</u> |
| $T_{ICL}$: | Terminator of the isocitrate lyase gene derived from <u>Corynebacterium</u> <u>glutamicum</u> |
| igk: | Inosine-guanosine kinase gene derived from <u>Escherichia</u> <u>coli</u> |
| Km$^r$: | Kanamycin resistance gene |
| Ap$^r$: | Ampicillin resistance gene |
| E.coli ORI: | Replication origin of <u>Escherichia</u> <u>coli</u> |

<u>Detailed Description of the Invention</u>

The present invention provides a process wherein the fermentation step to produce a precursor of a purine nucleotide by direct fermentation and the reaction step to enzymatically convert the precursor produced in said fermentation step into the purine nucleotide are carried out successively using a single microorganism in one fermentor.

The following three requirements have been found to be important for the establishment of said process.

The first requirement is that in the fermentation step to produce the precursor of the purine nucleotide by direct fermentation, the expression of a gene encoding an enzyme capable of synthesizing the purine nucleotide from its precursor needs to be suppressed to such a level that the fermentation is not substantially disturbed.

If the expression of said enzyme can not be suppressed, the purine nucleotide is formed and accumulated intracellularly by the activity of said enzyme during the fermentation and the fermentation yield of the purine nucleotide falls for the following reasons.

When GMP is accumulated as the purine nucleotide, the feedback inhibition of the key enzyme in the purine biosynthetic pathway is induced, whereby the production of XMP stops. When IMP is accumulated, a futile cycle is formed with inosine formation, causing waste of ATP. Thus, intracellular formation and accumulation of the purine nucleotide lowers the fermentation yield, which leads to an economical disadvantage.

The second requirement is that in the reaction step to synthesize the purine nucleotide from its precursor, it is needed to sufficiently induce the activity of the enzyme capable of synthesizing the purine nucleotide from its precursor.

The third requirement is that a producing microorganism which has undergone the fermentation and the induction treatment needs to retain a sufficient ATP-regenerating activity and the phosphorylation or amination activity for the conversion reaction, and these activities need to fully function in the reaction step to synthesize the purine nucleotide from its precursor.

The microorganism to be used in the present invention may be a wild strain, a mutant, a fused cell line, a transductant or a recombinant strain obtained by means of recombinant DNA techniques, so long as it has the ability to produce a precursor of a purine nucleotide and has the ATP-regenerating activity. Preferred microorganisms include those belonging to the genus <u>Corynebacterium</u>, <u>Escherichia</u> or <u>Bacillus</u>, which are useful for nucleic acid fermentation and amino acid fermentation. Particularly preferred is <u>Corynebacterium</u> <u>ammoniagenes</u> which has a strong ATP-regenerating activity.

The precursors of purine nucleotides include XMP, guanosine, inosine, adenosine, etc.

The ATP-regenerating activity refers to the activity to regenerate ATP from AMP or ADP using inorganic phosphoric acid in the process where a microorganism metabolizes sugars as energy donor substrates. This ATP-regenerating system is a part of the carbohydrate metabolism system and the energy metabolism system such as the glycolytic pathway, the TCA cycle and the electron transport system, and almost all microorganisms have this activity.

Specific examples of the microorganisms to be used in the present invention include the following strains and mutants derived therefrom.

Corynebacterium ammoniagenes ATCC 6872
Corynebacterium ammoniagenes ATCC 21295
Corynebacterium ammoniagenes ATCC 21477
Corynebacterium glutamicum ATCC 13032
Corynebacterium glutamicum ATCC 14067
Corynebacterium glutamicum ATCC 13869
Escherichia coli ATCC 14948
Escherichia coli ATCC 11303
Escherichia coli ATCC 9637
Bacillus subtilis ATCC 14618

As the enzyme capable of synthesizing a purine nucleotide from its precursor to be used in the present invention, any enzyme capable of synthesizing a purine nucleotide from its precursor can be used, and suitable examples include XMP aminase, inosine-guanosine kinase, phosphatase and adenylate kinase.

Examples of the DNAs encoding the above enzymes are given below.

Genes encoding XMP aminase include those derived from Escherichia coli [Nucleic Acids Res., 13, 1303 (1985)], Bacillus subtilis [Nueleic Acids Res., 18, 6710 (1990)], Corynebacterium ammoniagenes (Genbank accession no. g2765074), etc.

Genes encoding inosine-guanosine kinase include those derived from Escherichia coli (WO91/08286), Exiguobacterium acetylicum (WO96/30501), etc.

Genes encoding phosphatase include those derived from Morganella morganii (Japanese Published Unexamined Patent Application No. 37785/97, Japanese Published Unexamined Patent Application No. 201481/98), etc.

Genes encoding adenylate kinase include that derived from Saccharomyces cerevisiae (J. Biol. Chem., 263, 19468 (1988)].

The genes encoding the enzymes capable of synthesizing a purine nucleotide from its precursor can be obtained by a known method.

For example, in the case of the gene encoding XMP aminase, primers are synthesized based on the sequences at both ends of the sequence of an XMP aminase structural gene, and the XMP aminase structural gene can be obtained by the polymerase chain reaction method (PCR method) using the prepared primers and the Escherichia coli chromosomal DNA, the Bacillus subtilis chromosomal DNA or the Corynebacterium ammoniagenes chromosomal DNA.

In the case of the gene encoding inosine-guanosine kinase, primers are synthesized based on the sequences at both ends of the sequence of an inosine-guanosine kinase structural gene, and the inosine-guanosine kinase structural gene can be obtained by the PCR method using the prepared primers and the Escherichia coli chromosomal DNA or the Exiguobacterium acetylicum chromosomal DNA. Similarly, by the use of the PCR method, a phosphatase structural gene can be obtained from the Morganella morganii chromosomal DNA and an adenylate kinase structural gene can be obtained from the Saccharomyces cerevisiae chromosomal DNA.

The thus obtained gene encoding the enzyme capable of synthesizing a purine nucleotide from its precursor is inserted into an appropriate inducible-expression vector so that the gene is under the control of the transcription and translation signal capable of regulating its induction and expression, whereby a recombinant plasmid which can regulate the expression of said gene can be obtained.

There is no specific restriction as to the inducible-expression vector, so long as it is replicable in a microorganism to be used and fulfills the following three conditions.

First, the expression of the gene encoding the enzyme capable of synthesizing a purine nucleotide from its precursor can be repressed to a level where fermentation is not substantially disturbed in the fermentation step to produce the precursor of the purine nucleotide by direct fermentation.

Secondly, the activity of the enzyme capable of synthesizing the purine nucleotide from its precursor is sufficiently induced by the induction treatment to be carried out prior to the reaction step to synthesize the purine nucleotide from its precursor.

Thirdly, said induction method is a method which allows a producing microorganism to retain a sufficient ATP-regenerating activity and the converting activity after the induction treatment.

Examples of the vectors which fulfill the above three conditions include pPAC31 which contains the $P_L$ promoter/cl857 repressor gene and is induced at a high temperature (WO98/12343), pBB1 which contains the lac promoter/lacIts repressor gene and is induced at a high temperature (Gene, 70, 415 (1988)], pRK248cIts which contains the $P_L$ promoter/cI857 repressor gene and is induced at a high pH (Gene, 97, 125 (1991)], pOSEX2 which contains the proU expression-regulating region and is induced under a high osmotic pressure (Gene, 151, 137 (1994)], pTrc99A which contains the trc promoter/lacI$^q$ repressor gene and is induced with isopropyl-β-D-thiogalactoside (IPTG) [Gene, 69, 301 (1988)], pAL9181 which contains the araBAD promoter/araC repressor gene and is induced with L-arabinose [Appl. Microbiol. Biotechnol., 37, 205 (1992)] and vectors induced by changing the carbon source from sugars to non-sugars. These vectors are inducible-expression vectors constructed for Escherichia coli.

Further, vectors obtained by modifying the transcription-translation signal region related to the regulation of the expression in the above inducible-expression vectors by mutation techniques or recombinant DNA techniques can be used as well.

When these known inducible-expression vectors or further improved vectors are used in host cells other than Escherichia coli, a replication origin which functions in the host is inserted therein. For example, when Corynebacterium ammoniagenes is employed as a host, a replication origin of a plasmid derived from Corynebacterium glutamicum which functions in Corynebacterium ammoniagenes or the like is inserted into said vector.

The plasmids derived from Corynebacterium glutamicum include pCG1 (Japanese Published Unexamined Patent Application No. 134500/82), pCG2 (Japanese Published Unexamined Patent Application No. 35197/83), pCG4 (Japanese Published Unexamined Patent Application No. 183799/82), PAM330 (Japanese Published Unexamined Patent Application No. 67699/83), and pAG1, pAG3, pAG14 and pAG50 (Japanese Published Unexamined Patent Application No. 166890/87).

The preferred vectors functioning in Corynebacterium include a plasmid vector capable of being induced by raising the temperature which is prepared by ligating the $P_L$ promoter/cI857 gene carried by pPAC31 which is a vector induced at a high temperature in Escherichia coli with a vector autonomously replicable in Corynebacterium, vector pCEX2 capable of being induced with a non-sugar carbon source such as acetic acid (Japanese Published Unexamined Patent Application No. 224259/91), etc.

On the other hand, when the transcription-translation signal related to the regulation of gene expression in the inducible-expression vectors does not function in a microorganism to be used, or when it functions but the above three requirements are not sufficiently fulfilled, it is necessary to improve said transcription-translation signal region by mutation techniques or recombinant DNA techniques so that the above three requirements can be fulfilled, or to develop a new system of induction and expression applicable to said microorganism. In the former case, the improvement can be carried out, for example, by site-directed mutagenesis referring to a transcription-translation signal sequence known for the microorganism to be used. In the latter case, the new system can be developed and constructed according to the method of developing the above general inducible-expression systems constructed for Escherichia coli, etc.

Preferred examples of the microorganisms carrying the inducible-expression system include Corynebacterium ammoniagenes strains, etc., which were respectively developed using, as the gene-inducing and expressing system, a temperature-inducible plasmid vector which is prepared by ligating the $P_L$ promoter/cI857 gene carried by pPAC31 which is a vector induced at a high temperature in Escherichia coli with a vector autonomously replicable in Corynebacterium, and pCEX2 capable of regulating the expression by addition of inexpensive acetic acid.

In the present invention, the gene encoding the enzyme capable of synthesizing a purine nucleotide from its precursor may exist in a vector plasmid or may be integrated into the chromosome of a host microorganism, so long as the above three requirements are fulfilled. That is, either a strain carrying a plasmid containing the gene or a strain in which the gene has been integrated into the chromosome can be used, so long as the expression of said gene can be appropriately regulated.

The inducible-expression plasmid containing the gene encoding the enzyme capable of synthesizing a purine nucleotide from its precursor can be introduced into a microorganism which produces the precursor of the purine nucleotide by using the protoplast method, the electric pulse method, the calcium chloride method, and conventional methods described in Molecular Cloning, A Laboratory Manual, Second Edition, Cold Spring Harbor Laboratory Press (1989) (hereinafter abbreviated as Molecular Cloning, Second Edition), etc.

For example, when a bacterium belonging to the genus Corynebacterium is used as the host microorganism, the protoplast method (Japanese Published Unexamined Patent Application No. 183799/82) and the electric pulse method (Japanese Published Unexamined Patent Application No. 207791/90) are particularly effective. When Escherichia coli is used as the host microorganism, the calcium chloride method [J. Mol. Biol., 53, 159 (1970)], etc. can also be employed.

The enzyme capable of synthesizing a purine nucleotide from its precursor can be induced and expressed by culturing the thus obtained transformant of the present invention carrying the introduced gene encoding the enzyme capable of synthesizing the purine nucleotide from its precursor in an ordinary medium containing carbon sources, nitrogen sources and inorganic substances, and additionally, trace organic nutrients, as may be required, to form and accumulate the precursor of the purine nucleotide, and then subjecting the resulting culture to the induction treatment such as heating or addition of acetic acid.

As the carbon source in the medium to be used in the above fermentation step, any carbon source which can be assimilated by said microorganism can be used. Examples of suitable carbon sources are carbohydrates such as glucose, fructose, sucrose, molasses, blackstrap molasses and starch hydrolyzate, alcohols such as ethanol, glycerin and sorbitol, organic acids such as pyruvic acid and lactic acid, and amino acids such as glycine, alanine, glutamic acid and aspartic acid. The preferable concentration thereof is in the range of 5-30%.

Examples of the nitrogen sources include ammonia, various inorganic and organic ammonium salts such as ammonium chloride, ammonium sulfate, ammonium nitrate, ammonium carbonate, ammonium acetate and ammonium phosphate, urea, various amino acids, peptone, NZ amine, meat extract, yeast extract, corn steep liquor, casein hydrolyzate, fish meal and digested matters thereof.

Examples of the inorganic substances include potassium dihydrogenphosphate, dipotassium hydrogenphosphate, magnesium sulfate, magnesium phosphate, sodium chloride, ferrous sulfate, manganese sulfate, zinc sulfate and calcium carbonate.

When the microorganism employed requires particular nutrients such as amino acids, nucleic acids and vitamins for the growth, these substances are added to the medium in appropriate amounts.

Culturing is carried out under aerobic conditions, for example, by shaking culture or spinner culture under aeration. The optimum temperature is usually 26-37°C. The culturing period is usually 1-5 days.

The induction treatment for the expression of the gene encoding the enzyme capable of synthesizing a purine nucleotide from its precursor is carried out under conditions appropriate for the vector used; for example, the vectors mentioned below are preferably treated under the following conditions.

| | |
|---|---|
| pPAC31 or pBB1: | spinner culture under aeration at 37-42°C for 1-24 hours |
| pRK248clts: | for 1-24 hours at pH 9 |
| pOSEX2: | for 1-24 hours in the presence of 50-300 mmol/l NaCl |
| pTrc99A: | for 1-24 hours in the presence of 0.1-0.5 mmol/l IPTG |
| pAL9181: | for 1-24 hours with addition of 0.2% L-arabinose |
| pCEX2: | for 1-24 hours with addition of 0.1-2% ammonium acetate or sodium acetate |

It is preferred to add a surfactant in the reaction step to synthesize a purine nucleotide from its precursor.

As the surfactant, those which promote the permeation of a purine nucleotide through a cell membrane are preferred. Suitable examples are cationic surfactants such as polyoxyethylene stearylamine (e.g., Nymeen S-215, NOF Corporation), cetyltrimethylammonium bromide, Cation FB and Cation F2-40E, anionic surfactants such as sodium oleylamide sulfate, Newrex TAB and Rapizole 80, and amphoteric surfactants such as polyoxyethylene sorbitan monostearate (e.g., Nonion ST221). The surfactant is usually used at a concentration of 0.1-50 mg/ml, preferably 1-20 mg/ml.

The reaction in the step to synthesize a purine nucleotide from its precursor is carried out at pH 6-8 at 20-40°C for 1-48 hours.

After the reaction is completed, the purine nucleotide formed and accumulated in the reaction mixture can be recovered by known methods such as a method comprising removing microbial cells and crystallizing the nucleotide by concentration, the activated carbon treatment and the ion exchange resin method [Isao Endo, et al., Kagakukogakkai (The Society of Chemical Engineers, Japan) (ed.), Bioseparation Process Binran (Handbook of Bioseparation Process), Kyoritsu Shuppan (1996)].

Certain embodiments of the present invention are illustrated in the following examples, which are not intended to limit the scope of the present invention. The gene engineering procedures were carried out according to the methods described in Molecular Cloning, Second Edition, unless otherwise specified.

Example 1 Construction of an XMP-Producing Strain of Corynebacterium ammoniagenes Carrying the XMP Aminase Gene of Escherichia coli under the Control of $P_L$ Promoter and Production of GMP by the Strain

(1) Construction of Inducible-Expression Plasmid pLAC857 Capable of Regulating the Expression of the XMP Aminase Gene by the Shift in Temperature

A thermal inducible-expression plasmid pLAC857 was constructed from a known plasmid pPLA66 through the following two steps. pPLA66 is a plasmid which expresses XMP aminase at a high level and which was constructed by ligating an XMP aminase gene at a position downstream of the $P_L$ promoter and the SD sequence of trpL [Biosci. Biotech. Biochem., 61, 840 (1997)].

A temperature sensitive repressor gene cI857 was inserted into plasmid pPLA66 in the following manner.

Plasmid pPLA66 (1 μg) was cleaved with EcoRI (5 units) and BglII (5 units). The cleaved fragments were separated by agarose gel electrophoresis, and a DNA fragment containing the $P_L$ promoter and the region of the XMP aminase gene (guaA gene) derived from Escherichia coli was recovered from the gel using QIAEXII (QIAGEN CO., LTD.).

Plasmid pPAC31 carrying the cI857 gene (WO98/12343) (1 µg) was cleaved with EcoRI (5 units) and BamHI (5 units). The cleaved fragments were separated by agarose gel electrophoresis, and a DNA fragment containing the cI857 gene, an ampicillin resistance gene and the replication origin derived from Escherichia coli was recovered from the gel.

The DNA fragment containing said gene and the previously prepared DNA fragment containing the guaA gene were ligated by the use of a ligation kit (Takara Shuzo Co., Ltd.).

Escherichia coli JM109 (Takara Shuzo Co., Ltd.) was transformed using the ligation mixture and then spread on L agar medium containing 100 µg/ml ampicillin to obtain an ampicillin-resistant transformant.

A plasmid DNA was extracted from the obtained transformant by the alkali bacteriolysis method.

The plasmid was cleaved with various restriction enzymes and analyzed, whereby it was confirmed that plasmid pLA857 comprising the XMP aminase gene, the cI857 gene, the ampicillin resistance gene and the replication origin of the plasmid autonomously replicable in Escherichia coli in the region under the control of the $P_L$ promoter was obtained.

The replication origin of the plasmid autonomously replicable in Corynebacterium was inserted into plasmid pLA857 in the following manner.

Plasmid pLA857 (1 µg) was cleaved with PstI (5 units) at the only PstI-cleavage site existing thereon. The cleaved fragment was separated by agarose gel electrophoresis and recovered from the gel, whereby a PstI-cleaved fragment of pLA857 was obtained.

Plasmid vector pCG116 replicable in Corynebacterium ammoniagenes (Japanese Published Unexamined Patent Application No. 265892/89) (1 µg) was cleaved with PstI (5 units). After dephosphorylation of the DNA was effected by alkali phosphatase treatment to prevent rebinding, extraction with phenol and precipitation with ethanol were carried out, whereby a PstI-cleaved fragment of pCG116 was obtained.

The above-obtained PstI-cleaved fragment of pLA857 and PstI-cleaved fragment of pCG116 were ligated using the above ligation kit.

Corynebacterium ammoniagenes ATCC 6872 was transformed using 1 µg of the DNA obtained by the ligation treatment by means of the electric pulse method [Appl. Microbiol. Biotechnol., 30, 283 (1989)] and then spread on A agar medium [0.5% glucose, 1% peptone, 0.5% meat extract, 0.5% yeast extract, 0.25% sodium chloride, 1.5% agar, 10 mg/l adenine and 10 mg/l guanine (pH 7.2)] containing 100 µg/ml spectinomycin.

A plasmid DNA was extracted from the obtained spectinomycin-resistant transformant by the alkali bacteriolysis method.

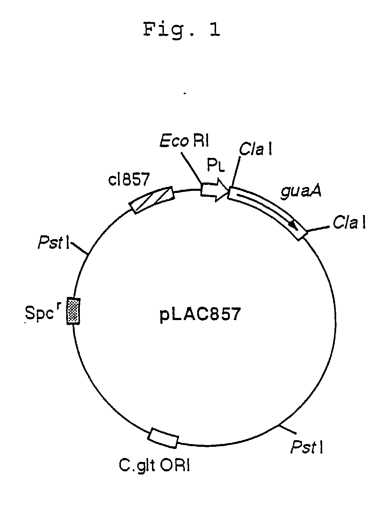

The plasmid was cleaved with various restriction enzymes and analyzed, whereby it was confirmed that the obtained transformant carried plasmid pLAC857 having the desired structure shown in Fig. 1.

Corynebacterium ammoniagenes ATCC 6872/pLAC857 carrying pLAC857 was deposited with the National Institute of Bioscience and Human-Technology, Agency of Industrial Science and Technology, 1-3, Higashi 1-chome, Tsukuba-shi, Ibaraki-ken, 305-0046 Japan, on February 5, 1999 with accession number FERM BP-6639 under the Budapest Treaty.

(2) Measurement of the XMP Aminase Activity of a Strain Prepared by Introducing pLAC857 into FERM BP-1261 Strain

Plasmid pLAC857 was extracted from Corynebacterium ammoniagenes ATCC 6872/pLAC857 (FERM BP-6639) by the alkali bacteriolysis method.

An XMP-producing strain of Corynebacterium ammoniagenes, FERM BP-1261 (Japanese Patent No. 2618383: adenine-leaky requiring and guanine-requiring), was transformed using said plasmid by the above electric pulse method.

A plasmid DNA was extracted from the obtained spectinomycin-resistant transformant by the alkali bacteriolysis method.

The plasmid was cleaved with various restriction enzymes and analyzed, whereby it was confirmed that the obtained transformant carried pLAC857.

The XMP-producing strain FERM BP-1261 carrying pLAC857 was cultured by shaking in A medium (a medium prepared by eliminating agar from A agar medium) containing 100 µg/ml spectinomycin at 30°C and 37°C, respectively for 24 hours.

After the culturing was completed, cells were obtained from each culture by centrifugation.

The obtained cells were washed twice with 100 mmol/l Tris-HCl (pH 7.0) and suspended in 10 ml of the same buffer.

To the resulting suspension was added 10 g of glass beads (Shinmaru Enterprises Co., Ltd., 0.1-0.2Ø), followed by disruption using a homogenizer (Nippon Seiki Co., Ltd.) under ice-cooling for 10 minutes.

The obtained suspension was centrifuged (14000 x g) at 4°C for 10 minutes, and the supernatant was recovered as a cell extract.

To 1.15 ml of a reaction mixture [160 mmol/l Tris-HCl (pH 8.6), 12 mmol/l ATP $Na_2 \cdot 3H_2O$, 16 mmol/l $MgSO_4 \cdot 7H_2O$ and 40 mmol/l $(NH_4)_2SO_4$] containing the cell extract previously heated to 42°C was added 0.1 ml of 0.3 mol/l XMP to start reaction at 42°C. Fifteen minutes later, 3.9 ml of 3.5% perchloric acid was added to the reaction mixture to stop the reaction.

The reaction mixture was centrifuged at 3000 rpm for 10 minutes, and the absorbance of the supernatant at 290 nm was measured. The specific activity per milligram of protein was calculated taking the activity to form 1 μmol of GMP in one minute as one unit (U). The amount of protein was determined using a protein assay kit (BioRad Laboratories).

The results are shown in Table 1. No activity was detected in the cells cultured at 30°C, while the activity was detected in the cells cultured at 37°C. These results indicate that the expression of the XMP aminase gene is under the control of the $P_L$ promoter in FERM BP-1261 strain carrying pLAC857, and the activity of said enzyme can be regulated by shifting the temperature.

Table 1

| Culturing temperature | Specific activity (μmol/min/mg protein) |
|---|---|
| 30°C | Not detected |
| 37°C | 0.25 |

(3) Production of GMP by the XMP-Producing Strain FERM BP-1261 Carrying pLAC857

FERM BP-1261 strain carrying pLAC857 was cultured on A agar medium containing 20 μg/ml streptomycin at 30°C for 2 days. The obtained cells were inoculated into a 250-ml Erlenmeyer flask containing 60 ml of C seed medium [5% glucose, 1% yeast extract, 1% peptone, 0.25% NaCl, 0.3% urea, 150 mg/l adenine and 150 mg/l guanine (pH 7.2)] containing 20 μg/ml streptomycin, followed by shaking culture at 30°C for 24 hours.

The whole of the obtained culture was inoculated into a 2-l fermentor containing 0.94 l of D seed medium [8.8% glucose, 1.7% meat extract, 1.7% peptone, 0.167% $KH_2PO_4$, 0.167% $K_2HPO_4$, 0.167% $MgSO_4 \cdot 7H_2O$, 333 mg/l adenine, 350 mg/l guanine, 33 mg/l $FeSO_4 \cdot 7H_2O$, 17 mg/l $ZnSO_4 \cdot 7H_2O$, 6.7 mg/l $MnSO_4 \cdot 4\text{-}6H_2O$, 25 mg/l β-alanine, 33 mg/l L-cysteine, 167 μg/l biotin, 1.3 mg/l $CuSO_4 \cdot 5H_2O$ and 8.3 mg/l thiamine (pH 7.2)], followed by culturing with stirring (600 rpm) and aeration (1 l/min) at 30°C for 24 hours, during which the pH was kept at 7.2 with 5.5 mol/l aqueous ammonia.

The obtained culture (120 ml) was inoculated into a 2-l fermentor containing 0.88 l of F fermentation medium [8% glucose, 1.67% orthophosphoric acid, 1.29% KOH, 1.2% $MgSO_4 \cdot 7H_2O$, 123mg/l $CaCl_2 \cdot 2H_2O$, 24.6 mg/l $FeSO_4 \cdot 7H_2O$, 12.3 mg/l $MnSO_4 \cdot 4\text{-}6H_2O$, 12.3mg/l $ZnSO_4 \cdot 7H_2O$, 18.5mg/l β-alanine, 24.6 mg/l L-cysteine, 6.2 mg/l nicotinamide, 24.6 mg/l histidine, 185 μg/l biotin, 2.5 mg/l $CuSO_4 \cdot 5H_2O$, 203 mg/l adenine and 10mg/l guanine (pH 7.2)], followed by culturing with stirring (600 rpm) and aeration (1 l/min) at 28°C for 44 hours, during which the pH was kept at 7.2 with 5.5 mol/l aqueous ammonia.

After the culturing was completed, the amounts of XMP and GMP accumulated in the culture supernatant were determined by HPLC in the following manner.

Conditions for HPLC analysis

Column:     Asahipak GS-320H (Asahi Chemical Industry Co., Ltd.)
Eluent:     0.2 mol/l $NaH_2PO_4$ (pH 3.0)
Flow rate:  1 ml/min
Detection:  UV 254 nm

The amounts of XMP and GMP accumulated were determined by measuring the absorbance at UV 254 nm and comparing the absorbance with the standard.

As the result of HPLC analysis, it was found that XMP was formed and accumulated in an amount of 27.2 g/l, but GMP was not detected.

To induce and express the XMP aminase gene, the culture broth was heated to 40°C, and kept at the same temperature with stirring (600 rpm) and aeration (1 l/min) for 6 hours, during which the pH was kept at 7.2 with 5.5 mol/l aqueous ammonia.

To the XMP fermentation broth subjected to the above thermal induction treatment were added 2.5% glucose, 10 g/l phytic acid, 4.4 g/l $MgSO_4 \cdot 7H_2O$, 9.36 g/l $Na_2HPO_4$, 96.9 mg/l adenine and 10 g/l Nymeen S-215, and the resulting mixture was subjected to reaction with stirring (600 rpm) and aeration (1 l/min) at 40°C for 24 hours, during which the pH was kept at 7.4 with 5.5 mol/l aqueous ammonia.

After the reaction was completed, the amount of GMP accumulated in the supernatant of the reaction mixture was determined under the above HPLC analysis conditions. As the result, it was found that GMP was formed and accumulated in an amount of 20.8 g/l.

Example 2 Construction of an XMP-Producing Strain of Corynebacterium ammoniagenes Carrying the XMP Aminase Gene of Corynebacterium ammoniagenes under the Control of the Promoter of an Isocitrate Lyase Gene and Production of GMP by the Strain

(1) Amplification and Cloning of the XMP Aminase Gene by PCR

Isolation of the XMP aminase gene of Corynebacterium ammoniagenes was carried out by PCR based on the nucleotide sequence in the following manner.

First, the oligonucleotides shown in SEQ ID NOS: 1 and 2 which are located at both ends of the XMP aminase gene and which respectively have the cleavage sites for the restriction enzymes AflII and BamHI were synthesized. The chromosomal DNA of Corynebacterium ammoniagenes ATCC 6872 to be used as the template was prepared according to the method for preparing protoplasts by treating cells cultured in the presence of glycin with lysozyme and achromopeptidase (Japanese Published Unexamined Patent Application No. 225776/94).

The obtained chromosomal DNA (0.1 μg), the above oligonucleotides (0.25 μmol/l each) as the primers and Taq DNA polymerase (Takara Shuzo Co., Ltd., 2.5 units) were added to 0.1 ml of 10 mmol/l Tris-HCl buffer (pH 8.3) comprising 200 μmol/l each of dATP, dCTP, dGTP and dTTP, 50 mmol/l potassium chloride, 1.5 mmol/l magnesium chloride and 0.0001% gelatin, and the resulting mixture was subjected to PCR.

PCR was carried out by repeating 10 times a reaction cycle in which reaction is conducted at 94°C for 90 seconds, at 50°C for 120 seconds and at 72°C for 120 seconds; repeating 10 times a reaction cycle in which reaction is conducted at 94°C for 90 seconds, at 40°C for 120 seconds and at 72°C for 120 seconds; and further repeating 20 times a reaction cycle in which reaction is conducted at 94°C for 90 seconds, at 40°C for 120 seconds and at 72°C for 180 seconds; and then conducting reaction at 72°C for 360 seconds.

The obtained reaction mixture was subjected to agarose gel electrophoresis, whereby the desired DNA fragment of about 1.6 kb was recovered.

Said DNA fragment was cleaved with the restriction enzymes AflII (5 units) and BamHI (5 units), and a cleaved fragment of about 1.6 kb whose ends were respectively treated with AflII and BamHI was isolated and recovered by agarose gel electrophoresis.

(2) Construction of Inducible-Expression Plasmid pGUA2 Capable of Regulating the Expression of the XMP Aminase Gene with a Carbon Source

A plasmid which can regulate the expression of the XMP aminase gene with a carbon source was constructed in the following manner using inducible-expression vector pCEX2 (Japanese Published Unexamined Patent Application No. 224259/91).

pCEX2 is a vector containing the expression-regulating region and the transcription termination signal sequence of the isocitrate lyase gene of Corynebacterium glutamicum, and the expression of an exogenous gene inserted into the multicloning site thereon is suppressed in the presence of a sugar and is induced in the presence of a non-sugar such as acetic acid.

pCEX2 vector DNA (1 μg) was partially cleaved with AflII (0.5 unit) and then cleaved with BamHI (5 units). Then, a DNA fragment of 7.6 kb containing the expression-regulating region and the transcription termination signal region of the isocitrate lyase gene, a spectinomycin resistance gene and the replication origin of the plasmid autonomously replicable in Corynebacterium glutamicum was isolated and recovered by agarose gel electrophoresis.

Said DNA fragment and the PCR-amplified fragment of the XMP aminase gene obtained in the above Example 2 (1) were ligated to obtain a ligated DNA.

Corynebacterium ammoniagenes ATCC 6872 was transformed using said ligated DNA (1 μg) by the electric pulse method, and then spread on A agar medium containing 100 μg/ml spectinomycin.

A plasmid DNA was extracted from the obtained spectinomycin-resistant transformant by the alkali bacteriolysis method.

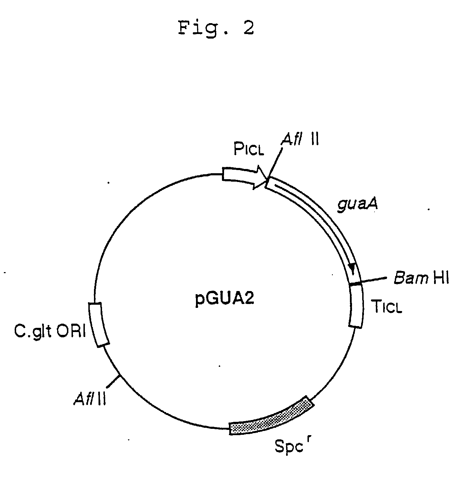

The plasmid DNA was cleaved with various restriction enzymes and analyzed, whereby it was confirmed that the obtained transformant carried plasmid pGUA2 having the desired structure shown in Fig. 2.

(3) Measurement of the XMP Aminase Activity of a Strain Prepared by Introducing pGUA2 into FERM BP-1261 Strain

Plasmid pGUA2 was extracted from Corynebacterium ammoniagenes ATCC 6872 carrying pGUA2 by the alkali bacteriolysis method.

An XMP-producing strain of Corynebacterium ammoniagenes, FERM BP-1261 (adenine-leaky requiring and guanine-requiring), was transformed using said plasmid by the electric pulse method.

A plasmid DNA was extracted from the obtained spectinomycin-resistant transformant by the alkali bacteriolysis method.

The plasmid was cleaved with various restriction enzymes and analyzed, whereby it was confirmed that the transformant carried pGUA2.

The XMP-producing strain FERM BP-1261 carrying pGUA2 was cultured by shaking in two kinds of media, i.e., A medium containing 100 μg/ml spectinomycin and a medium prepared by replacing glucose in said medium by ammonium acetate, separately, at 30°C for 24 hours.

Cells were obtained from the resulting cultures to prepare cell extracts in the same manner as in Example 1 (2).

The XMP aminase activity in each of the extracts was measured according to the method described in Example 1 (2).

The results are shown in Table 2.

Only an extremely low level of activity was detected in the cells cultured using glucose as the carbon source, while a high level of activity was detected in the cells cultured using 2% ammonium acetate as the carbon source.

These results indicate that the expression of the XMP aminase gene is under the control of the promoter of the isocitrate lyase gene in FERM BP-1261 carrying pGUA2, and the activity of said enzyme can be regulated by changing the carbon source.

Table 2

| Carbon source | Specific activity (μmol/min/mg protein) |
|---|---|
| Glucose | 0.05 |
| Ammonium acetate | 0.38 |

(4) Production of GMP by the Strain Prepared by Introducing pGUA2 to XMP-Producing Strain FERM BP-1261

XMP fermentation was carried out using FERM BP-1261 strain carrying pGUA2 under the same culturing conditions as in Example 1 (3). After the culturing was completed, the amounts of XMP and GMP formed and accumulated in the culture supernatant were determined in the same manner as in Example 1 (3).

As the result, the amount of XMP accumulated was found to be 18.4 g/l. GMP was not detected.

After the fermentation was completed, in order to induce and express the XMP aminase gene, ammonium acetate was added to the fermentation broth to a final concentration of 2%, followed by culturing with stirring (600 rpm) and aeration (1 l/min) for 10 hours, during which the pH was kept at 7.2 with 5.5 mol/l aqueous ammonia.

To the culture subjected to the above induction treatment with ammonium acetate were added 2.5% glucose, 10 g/l phytic acid, 4.4 g/l $MgSO_4 \cdot 7H_2O$, 9.36 g/l $Na_2HPO_4$, 96.9 mg/l adenine and 10 g/l Nymeen S-215, and the resulting mixture was subjected to reaction with stirring (600 rpm) and aeration (1 l/min) at 40°C for 24 hours, during which the pH was kept at 7.4 with 5.5 mol/l aqueous ammonia.

After the reaction was completed, the amount of GMP accumulated in the supernatant of the reaction mixture was determined according to the method described in Example 1 (3).

It was found that GMP was formed and accumulated in an amount of 14.4 g/l in the reaction mixture.

Example 3 Construction of an Inosine-Producing Strain of Corynebacterium ammoniagenes Carrying the Inosine-Guanosine Kinase Gene of Escherichia coli under the Control of the Promoter of an Isocitrate Lyase Gene and Production of IMP by the Strain

(1) Amplification and Cloning of the Inosine-Guanosine Kinase Gene by PCR

Isolation of the inosine-guanosine kinase gene of Escherichia coli was carried out by PCR based on the known nucleotide sequence (WO91/08286) in the following manner.

The oligonucleotides shown in SEQ ID NOS: 3 and 4 which are located at both ends of the inosine-guanosine kinase gene and which respectively have the cleavage sites for the restriction enzymes AflII and BamHI were synthesized.

Plasmid pBM2 carried by Escherichia coli HM70/pBM2 (WO91/08286) was used as the template of the inosine-guanosine kinase gene.

The above plasmid DNA (0.1 μg), the above oligonucleotides (0.25 μmol/l each) as the primers and Taq DNA polymerase (Takara Shuzo Co., Ltd., 2.5 units) were added to 0.1 ml of 10 mmol/l Tris-HCl buffer (pH 8.3) comprising 200 μmol/l each of dATP, dCTP, dGTP and dTTP, 50 mmol/l potassium chloride, 1.5 mmol/l magnesium chloride and 0.0001% gelatin, and the resulting mixture was subjected to PCR.

PCR was carried out by repeating 10 times a reaction cycle in which reaction was conducted at 94°C for 90 seconds, at 50°C for 120 seconds and at 72°C for 120 seconds; repeating 10 times a reaction cycle in which reaction was conducted at 94°C for 90 seconds, at 40°C for 120 seconds and at 72°C for 120 seconds; further repeating 20 times a reaction cycle in which reaction was conducted at 94°C for 90 seconds, at 40°C for 120 seconds and at 72°C for 180 seconds; and then conducting reaction at 72°C for 360 seconds.

The obtained PCR reaction product was ligated with vector pT-Adv which is a vector for the cloning of a PCR product (Clontech Co., Ltd.).

Escherichia coli DH5α was transformed using 1 μg of the DNA obtained by said ligation treatment, and then spread on L agar medium containing 100 μg/ml ampicillin.

Plasmid pT-AI was extracted from the obtained ampicillin-resistant transformant by the alkali bacteriolysis method.

The structure of the plasmid pT-AI was analyzed using various restriction enzymes, whereby it was confirmed that this plasmid contained the inosine-guanosine kinase gene (1.3 kb), an ampicillin resistance gene, a kanamycin resistance gene and the replication origin of the plasmid autonomously replicable in Escherichia coli.

(2) Construction of Inducible-Expression Plasmid pIGK2 Capable of Regulating the Expression of the Inosine-Guanosine Kinase Gene with a Carbon Source

A plasmid which can regulate the expression of the inosine-guanosine kinase gene with a carbon source was constructed in the following manner using inducible-expression vector pCEX2 (Japanese Published Unexamined Patent Application No. 224259/91).

pCEX2 vector DNA (1 μg) was cleaved with KpnI (5 units) and a DNA fragment of 7.6 kb containing the expression-regulating region and the transcription termination signal region of the isocitrate lyase gene, a spectinomycin resistance gene and the replication origin of the plasmid autonomously replicable in Corynebacterium glutamicum was isolated and recovered by agarose gel electrophoresis.

The plasmid pT-AI containing the inosine-guanosine kinase gene obtained in Example 3 (1) (1 μg) was cleaved with KpnI (5 units), followed by dephosphorylation.

This KpnI-cleaved DNA fragment and the above KpnI-cleaved fragment of pCEX2 were ligated.

Escherichia coli DH5α was transformed using the obtained ligation mixture, and then spread on L agar medium containing 100 μg/ml ampicillin to obtain an ampicillin-resistant transformant.

A plasmid was extracted from the obtained transformant by the alkali bacteriolysis method.

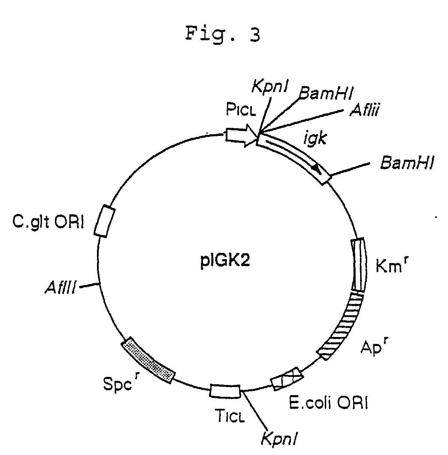

The plasmid was cleaved with various restriction enzymes and analyzed, whereby it was confirmed that plasmid pIGK2 having the desired structure shown in Fig. 3 was obtained.

Corynebacterium ammoniagenes ATCC 6872 was transformed using this plasmid (1 μg) by the electric pulse method and then spread on A agar medium containing 100 μg/ml spectinomycin.

A plasmid DNA was extracted from the obtained spectinomycin-resistant transformant by the alkali bacteriolysis method and it was confirmed that the transformant carried pIGK2.

Corynebacterium ammoniagenes ATCC 6872/pIGK2 carrying pIGK2 was deposited with the National Institute of Bioscience and Human-Technology, Agency of Industrial Science and Technology, 1-3, Higashi 1-chome, Tsukuba-shi, Ibaraki-ken, 305-0046 Japan, on February 5, 1999 with accession number FERM BP-6638 under the Budapest Treaty.

(3) Measurement of the Inosine-Guanosine Kinase Activity of a Strain Prepared by Introducing pIGK2 into FERM BP-2217 Strain

Plasmid pIGK2 was extracted from <u>Corynebacterium</u> <u>ammoniagenes</u> ATCC 6872/pIGK2 (FERM BP-6638) by the alkali bacteriolysis method.

An inosine-producing strain of <u>Corynebacterium</u> <u>ammoniagenes</u>, FERM BP-2217 (Japanese Patent No. 2578496: adenine-leaky requiring and guanine-requiring), was transformed using said plasmid by the electric pulse method.

A plasmid DNA was extracted from the obtained spectinomycin-resistant transformant by the alkali bacteriolysis method.

The plasmid was cleaved with various restriction enzymes and analyzed, whereby it was confirmed that the obtained transformant carried pIGK2.

The inosine-producing strain FERM BP-2217 carrying pIGK2 was cultured in two kinds of media, i.e., A medium containing 100 μg/ml spectinomycin and a medium prepared by replacing glucose in said medium by ammonium acetate, separately, at 30°C for 24 hours.

Cells were obtained from the resulting cultures to prepare cell extracts according to the method described in Example 1 (2).

The inosine-guanosine kinase activity in each of the extracts was measured in the following manner.

To 0.1 ml of a reaction mixture [100 mmol/l HEPES buffer (pH 7.2), 10 mmol/l $MgSO_4$, 50 mmol/l KCl, 1 mmol/l ATP and 1 mmol/l inosine] previously heated to 30°C was added 0.01 ml of the cell extract, followed by reaction at 30°C for about 30 minutes.

During the reaction, the reaction mixture was sampled at intervals and the samples taken were diluted 20-fold with 0.2 mol/l $NaH_2PO_4$ (adjusted to pH 2.6 with $H_3PO_4$) to stop the reaction.

The amounts of inosine and IMP in the diluted samples were determined by HPLC analysis.

Conditions for HPLC analysis

Column: Asahipak GS-320H (Asahi Chemical Industry Co., Ltd.)
Eluent: 0.2 mol/l $NaH_2PO_4$ (pH 2.6)
Flow rate: 1 ml/min
Detection: UV 254 nm

The amounts of inosine and IMP accumulated were determined by measuring the absorbance at UV 254 nm and comparing the absorbance with the standard. The specific activity per milligram of protein was calculated taking the activity to form 1 μmol of IMP in one minute as one unit (U).

The amount of protein was determined using a protein assay kit (BioRad Laboratories).

The results are shown in Table 3. Only an extremely low level of activity was detected in the cells cultured using glucose as the carbon source, while a high level of activity was detected in the cells cultured using 2% ammonium acetate as the carbon source. These results indicate that the expression of the inosine-guanosine kinase gene is under the control of the promoter of the isocitrate lyase gene in FERM BP-2217 carrying pIGK2, and the activity of said enzyme can be regulated by changing the carbon source.

Table 3

| Carbon source | Specific activity (μmol/min/mg protein) |
| --- | --- |
| Glucose | 0.02 |
| Ammonium acetate | 0.34 |

(4) Production of IMP by the Inosine-Producing Strain FERM BP-2217 Carrying pIGK2

FERM BP-2217 strain carrying pIGK2 was cultured on A agar medium containing 20 μg/ml streptomycin at 30°C for 2 days.

After the culturing was completed, the obtained cells were inoculated into a 250-ml Erlenmeyer flask containing 60 ml of CI seed medium [5% glucose, 1% yeast extract, 1% peptone, 0.25% NaCl, 0.25% urea, 300 mg/l adenine and 100 mg/l guanine (pH 7.2)] containing 20 $\mu$g/ml streptomycin, followed by shaking culture at 30°C for 24 hours.

The whole of the obtained culture was inoculated into a 2-l fermentor containing 0.94 l of DI seed medium [7% glucose, 1% meat extract, 1% peptone, 0.1% $KH_2PO_4$, 0.1% $K_2HPO_4$, 0.1% $MgSO_4 \cdot 7H_2O$, 300 mg/l adenine, 300 mg/l guanine, 10 mg/l $FeSO_4 \cdot 7H_2O$, 10 mg/l $ZnSO_4 \cdot 7H_2O$, 10 mg/l $MnSO_4 \cdot 4\text{-}6H_2O$, 16 mg/l $\beta$-alanine, 20 mg/l L-cysteine, 30 $\mu$g/l biotin, 2 mg/l $CuSO_4 \cdot 5H_2O$ and 6 mg/l thiamine (pH 7.2)], followed by culturing with stirring (600 rpm) and aeration (1 l/min) at 30°C for 24 hours, during which the pH was kept at 7.2 with 5.5 mol/l aqueous ammonia.

The obtained culture (120 ml) was inoculated into a 2-l fermentor containing 0.88 l of FI fermentation medium [8% glucose, 2.07% CSL, 0.21% $KH_2PO_4$, 0.21% $K_2HPO_4$, 0.43% $MgSO_4 \cdot 7H_2O$, 105 mg/l $CaCl_2 \cdot 2H_2O$, 10.4 mg/l $FeSO_4 \cdot 7H_2O$, 20.7 mg/l $MnSO_4 \cdot 4\text{-}6H_2O$, 5.2 mg/l $ZnSO_4 \cdot 7H_2O$, 10.4 mg/l calcium pantothenate, 20.7 mg/l L-cysteine, 5.2 mg/l nicotinic acid, 93.8 $\mu$g/l biotin, 0.51 mg/l $CuSO_4 \cdot 5N_2O$ and 313 mg/l adenine (pH 7.2)], followed by culturing with stirring (600 rpm) and aeration (1 l/min) at 28°C for 44 hours, during which the pH was kept at 7.2 with 5.5 mol/l aqueous ammonia.

After the culturing was completed, the amounts of inosine and IMP formed and accumulated in the culture supernatant were determined according to the method described in Example 3 (3).

The amount of inosine formed and accumulated in the culture supernatant was 23.1 g/l and IMP was not detected.

After the culturing was completed, in order to induce and express the inosine-guanosine kinase gene, ammonium acetate was added to the obtained culture to a final concentration of 2%, followed by culturing with stirring (600 rpm) and aeration (1 l/min) for 10 hours, during which the pH was kept at 7.2 with 5.5 mol/l aqueous ammonia.

To the obtained culture were added 2.5% glucose, 10 g/l phytic acid, 4.4 g/l $MgSO_4 \cdot 7H_2O$, 9.36 g/l $Na_2HPO_4$, 96.9 mg/l adenine and 10 g/l Nymeen S-215, and the resulting mixture was subjected to reaction with stirring (600 rpm) and aeration (1 l/min) at 40°C for 24 hours, during which the pH was kept at 7.4 with 5.5 mol/l aqueous ammonia.

After the reaction was completed, the amount of IMP accumulated in the supernatant of the reaction mixture was determined according to the method described in Example 3 (3).

It was found that IMP was formed and accumulated in an amount of 37.7 g/l in the reaction mixture.

**Claims**

1. A process for producing a purine nucleotide which comprises: culturing in a medium a microorganism having the ability to produce a precursor of the purine nucleotide and carrying an introduced DNA which can express an enzyme capable of synthesizing the purine nucleotide from said precursor upon induction; allowing said precursor of the purine nucleotide to accumulate in the culture; inducing the expression of the enzyme capable of synthesizing the purine nucleotide from said precursor and expressing said enzyme; allowing the purine nucleotide formed from said precursor to accumulate in said culture; and then recovering said purine nucleotide therefrom.

2. The process according to claim 1, wherein the precursor of the purine nucleotide is 5'-xanthylic acid, the enzyme capable of synthesizing the purine nucleotide from said precursor is 5'-xanthylic acid aminase, and the purine nucleotide is 5'-guanylic acid.

3. The process according to claim 1, wherein the precursor of the purine nucleotide is guanosine, the enzyme capable of synthesizing the purine nucleotide from said precursor is inosine-guanosine kinase or phosphatase, and the purine nucleotide is 5'-guanylic acid.

4. The process according to claim 1, wherein the precursor of the purine nucleotide is inosine, the enzyme capable of synthesizing the purine nucleotide from said precursor is inosine-guanosine kinase or phosphatase, and the purine nucleotide is 5'-inosinic acid.

5. The process according to any one of claims 1 to 4, wherein the microorganism belongs to the genus selected from the group consisting of Corynebacterium, Escherichia and Bacillus.

6. The process according to claim 5, wherein the microorganism is Corynebacterium ammoniagenes.

7. The process according to claim 6 wherein the microorganism is Corynebacterium ammoniagenes ATCC 6872/pLAC857 (FERM BP-6639) or Corynebacterium ammoniagenes ATCC 6872/pIGK2 (FERM BP-6638).

8. The process according to any one of claims 1 to 6, which is characterized in that the expression of the enzyme capable of synthesizing the purine nucleotide is induced by the change of condition selected from the group consisting of rise in temperature, rise in pH and rise in osmotic pressure, or by the change of the carbon source from sugars to non-sugars.

9. The process according to claim 8, wherein the non-sugar carbon source is acetic acid or acetate.

10. A DNA which can express an enzyme capable of synthesizing a purine nucleotide from its precursor upon induction.

11. The DNA according to claim 10, which can express the enzyme capable of synthesizing the purine nucleotide upon induction by the change of condition selected from the group consisting of rise in temperature, rise in pH and rise in osmotic pressure, or by the change of the carbon source from sugars to non-sugars.

12. The DNA according to claim 11, wherein the non-sugar carbon source is acetic acid or acetate.

**13.** The DNA according to any one of claims 10 to 12, which is the plasmid DNA contained in the microorganism deposited under the deposit numbers FERM BP-6639 or FERM BP-6638.

**14.** A microorganism having the ability to produce a precursor of a purine nucleotide and carrying an introduced DNA which can express an enzyme capable of synthesizing the purine nucleotide from said precursor upon induction.

**15.** The microorganism according to claim 14, wherein the precursor of the purine nucleotide is 5'-xanthylic acid, the enzyme capable of synthesizing the purine nucleotide from said precursor is 5'-xanthylic acid aminase, and the purine nucleotide is 5'-guanylic acid.

**16.** The microorganism according to claim 14, wherein the precursor of the purine nucleotide is guanosine, the enzyme capable of synthesizing the purine nucleotide from said precursor is inosine-guanosine kinase or phosphatase, and the purine nucleotide is 5'-guanylic acid.

**17.** The microorganism according to claim 14, wherein the precursor of the purine nucleotide is inosine, the enzyme capable of synthesizing the purine nucleotide from said precursor is inosine-guanosine kinase or phosphatase, and the purine nucleotide is 5'-inosinic acid.

**18.** The microorganism according to claim 14, which belongs to the genus selected from the group consisting of Corynebacterium, Escherichia and Bacillus.

**19.** The microorganism according to claim 18, which is Corynebacterium ammoniagenes.

**20.** The microorganism according to claim 19, which is Corynebacterium ammoniagenes ATCC 6872/pLAC857 (FERM BP-6639) or Corynebacterium ammoniagenes ATCC 6872/pIGK2 (FERM BP-6638).

Fig. 1

Fig. 2

Fig. 3

**European Patent Office**

**EUROPEAN SEARCH REPORT**

Application Number

EP 00 10 2645

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 236 716 A (ALLIED CORP) 16 September 1987 (1987-09-16) * the whole document * | 10,11 | C12P19/32 C12N15/63 C12N15/52 C12N15/54 |
| X | EP 0 251 489 A (KYOWA HAKKO KOGYO KK) 7 January 1988 (1988-01-07) * the whole document * | 10,11 | C12N15/55 C12N9/00 C12N9/12 C12N9/16 |
| X | EP 0 185 092 A (KYOWA HAKKO KOGYO KK) 25 June 1986 (1986-06-25) * the whole document * | 10,11 | C12N1/21 //(C12N1/21, C12R1:15) |
| D,X | T. FUJIO ET AL.: "High level expression of XMP aminase in Escherichia coli and its application for the industrial production of 5'-guanylic acid" BIOSCIENCE BIOTECHNOLOGY BIOCHEMISTRY, vol. 61, no. 5, May 1997 (1997-05), pages 840-845, XP002138862 TOKYO JP * the whole document * | 10,11 | |
| | | | **TECHNICAL FIELDS SEARCHED (Int.Cl.7)** |
| X | EP 0 458 971 A (KYOWA HAKKO KOGYO KK) 4 December 1991 (1991-12-04) * the whole document * | 10 | C12P C12N |
| X | EP 0 816 491 A (AJINOMOTO KK) 7 January 1998 (1998-01-07) * the whole document * | 10 | |
| X | H. MORI ET AL.: "A novel process of inosine 5'-monophosphate production using overexpressed guanosine/inosine kinase" APPLIED MICROBIOL. AND BIOTECHNOL., vol. 48, no. 6, December 1997 (1997-12), pages 693-698, XP000907475 SPRINGER INTERNATIONAL, NEW YORK, US * the whole document * | 10 | |

-/--

The present search report has been drawn up for all claims

| Place of search | Date of completion of the search | Examiner |
|---|---|---|
| THE HAGUE | 26 May 2000 | Hornig, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 00 10 2645

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | H. MORI ET AL.: "Cloning of a guanosine-inosine kinase gene of Escherichia coli and characterization of the purified gene product" J. BACTERIOL., vol. 177, no. 17, September 1995 (1995-09), pages 4921-4926, XP002138863 AM. SOC. MICROBIOL.,BALTIMORE,US; * the whole document * | 10 | |
| X | EP 0 832 970 A (AJINOMOTO KK) 1 April 1998 (1998-04-01) * the whole document * | 10 | |
| X | EP 0 857 788 A (AJINOMOTO KK) 12 August 1998 (1998-08-12) * the whole document * | 10 | |

**TECHNICAL FIELDS SEARCHED    (Int.Cl.7)**

The present search report has been drawn up for all claims

| Place of search | Date of completion of the search | Examiner |
|---|---|---|
| THE HAGUE | 26 May 2000 | Hornig, H |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

 

& : member of the same patent family, corresponding
   document

**ANNEX TO THE EUROPEAN SEARCH REPORT
ON EUROPEAN PATENT APPLICATION NO.**    EP 00 10 2645

This annex lists the patent family members relating to the patent documents cited in the above-mentioned European search report.
The members are as contained in the European Patent Office EDP file on
The European Patent Office is in no way liable for these particulars which are merely given for the purpose of information.

26-05-2000

| Patent document cited in search report | | Publication date | Patent family member(s) | | Publication date |
|---|---|---|---|---|---|
| EP 0236716 | A | 16-09-1987 | US | 4655532 A | 07-04-1987 |
| | | | JP | 62217582 A | 25-09-1987 |
| EP 0251489 | A | 07-01-1988 | JP | 1984640 C | 25-10-1995 |
| | | | JP | 7016431 B | 01-03-1995 |
| | | | JP | 62285797 A | 11-12-1987 |
| | | | DE | 3780527 A | 27-08-1992 |
| | | | DE | 3780527 T | 04-03-1993 |
| EP 0185092 | A | 25-06-1986 | JP | 60192597 A | 01-10-1985 |
| | | | JP | 60224498 A | 08-11-1985 |
| | | | DE | 3586741 A | 12-11-1992 |
| | | | DE | 3586741 T | 06-05-1993 |
| | | | WO | 8504187 A | 26-09-1985 |
| EP 0458971 | A | 04-12-1991 | DE | 69024639 D | 15-02-1996 |
| | | | DE | 69024639 T | 05-06-1996 |
| | | | JP | 2999256 B | 17-01-2000 |
| | | | AT | 132528 T | 15-01-1996 |
| | | | CA | 2046865 A | 06-06-1991 |
| | | | ES | 2084046 T | 01-05-1996 |
| | | | WO | 9108286 A | 13-06-1991 |
| | | | KR | 145418 B | 15-07-1998 |
| | | | US | 5756315 A | 26-05-1998 |
| EP 0816491 | A | 07-01-1998 | BR | 9607745 A | 23-06-1998 |
| | | | CA | 2216172 A | 03-10-1996 |
| | | | CN | 1185174 A | 17-06-1998 |
| | | | HU | 9801687 A | 28-10-1998 |
| | | | WO | 9630501 A | 03-10-1996 |
| | | | PL | 322580 A | 02-02-1998 |
| EP 0832970 | A | 01-04-1998 | JP | 9037785 A | 10-02-1997 |
| | | | PL | 323493 A | 30-03-1998 |
| | | | US | 6010851 A | 04-01-2000 |
| | | | CA | 2221774 A | 28-11-1996 |
| | | | CN | 1191566 A | 26-08-1998 |
| | | | HU | 9900808 A | 28-07-1999 |
| | | | WO | 9637603 A | 28-11-1996 |
| EP 0857788 | A | 12-08-1998 | JP | 10201481 A | 04-08-1998 |
| | | | BR | 9705813 A | 27-04-1999 |
| | | | CN | 1184157 A | 10-06-1998 |
| | | | US | 6015697 A | 18-01-2000 |

For more details about this annex : see Official Journal of the European Patent Office, No. 12/82